Europäisches Patentamt

European Patent Office

Office européen des brevets

(19)

(11) Veröffentlichungsnummer: **0 304 853 B1**

# EUROPÄISCHE PATENTSCHRIFT

(12)

(45) Veröffentlichungstag der Patentschrift: **13.01.93**

(51) Int. Cl.5: **C07C 45/41**, C07C 47/54, C07C 47/542, C07C 47/02

(21) Anmeldenummer: **88113657.6**

(22) Anmeldetag: **23.08.88**

(54) **Verfahren zur Herstellung von Aldehyden.**

(30) Priorität: **28.08.87 DE 3728764**

(43) Veröffentlichungstag der Anmeldung:
**01.03.89 Patentblatt 89/09**

(45) Bekanntmachung des Hinweises auf die
Patenterteilung:
**13.01.93 Patentblatt 93/02**

(84) Benannte Vertragsstaaten:
**AT BE DE ES FR GB IT NL**

(56) Entgegenhaltungen:
**EP-A- 0 178 718**
**DE-A- 3 819 436**

CHEMICAL ABSTRACTS, Band 93, 1980, Seite 103, Zusammenfassung Nr. 48586t, Columbus, Ohio, US; W. OSTERTAG et al.: "Formation of transparent iron oxide red by iron carbonyl combustion"

CHEMICAL ABSTRACTS, Band 101, 1984, Seite 557, Zusammenfassung Nr. 239239a, Columbus, Ohio, US; S. OKAMOTO et al.: "Highly-dispersed iron oxides prepared by photooxidation of iron pentacarbonyl
[Fe(CO)5] supported on silicon dioxide gel at low temperature. Formation of an x-ray amorphous iron oxide"

(73) Patentinhaber: **BASF Aktiengesellschaft Carl-Bosch-Strasse 38 W-6700 Ludwigshafen(DE)**

(72) Erfinder: **Wambach, Ludwig, Dr. Humboldtstrasse 28 W-6900 Heidelberg(DE)**
Erfinder: **Irgang, Matthias, Dr. Andreas-Hofer-Weg 41 W-6900 Heidelberg(DE)**
Erfinder: **Fischer, Martin, Dr. Elbinger Weg 1 W-6700 Ludwigshafen(DE)**

**Beschreibung**

Die vorliegende Erfindung betrifft ein verbessertes Verfahren zur Herstellung von Aldehyden durch Gasphasenhydrierung von aromatischen Carbonsäuren oder aliphatischen Carbonsäuren, die in $\alpha$-Stellung zur Carboxylgruppe höchstens ein Wasserstoffatom tragen, oder von Estern oder Anhydriden dieser Säuren mit Hilfe eines Eisenkatalysators.

Die Reduktion aromatischer und aliphatischer Carbonsäuren mit molekularem Wasserstoff zu den korrespondierenden Aldehyden mit Hilfe verschiedener Katalysatorsysteme ist bekannt. Gemäß der US-A 3 935 265 werden beispielsweise Wasserstoff und aromatische Ester mit Aluminiumoxid bei Temperaturen zwischen 400 und 500°C in Kontakt gebracht. Aus Methylbenzoat erhält man auf diesem Weg bei einem Umsatz von 39 % eine Selektivität für Benzaldehyd von 37 %.

Die Hydrierung von Carbonsäuren und Estern, welche keinen Wasserstoff am $\alpha$-C-Atom besitzen, wie Benzoesäure und Methylbenzoat, ist ferner in der US-A 4 328 373 beschrieben. Als Katalysatoren werden Oxide der Metalle Yttrium, Zirkon, Cer, Praseodym, Thorium und Uran auf $\alpha$-Aluminiumoxid verwendet. Die ersten beiden Oxide werden dabei bevorzugt. Im Falle der Hydrierung der Benzoesäure über einem Katalysator aus 98 % Zirkonoxid erzielt man einen Umsatz von 98,6 % bei einer Selektivität für Benzaldehyd von 64,7 %. Allerdings wurde der Versuch nach 21,7 Stunden abgebrochen und die Raumzeitausbeute ist sehr klein. Ein eigener Vergleichsversuch mit einem Katalysator der Zusammensetzung 93,2 % $ZrO_2$ und 6,8 % $Cr_2O_3$ zeigte bei 415°C allerdings eine weitaus geringere Aktivität, wie aus Beispiel 2 (Vergleichsbeispiel) zu entnehmen ist.

In der EP-A 0 191 995 wird gezeigt, daß man Carbonsäuren und deren Ester mit Manganoxidkatalysatoren zu den korrespondierenden Aldehyden hydrieren kann. Bei einem Umsatz an Benzoesäure von 91 % liefert dieser Katalysator Benzaldehyd mit einer Selektivität von 95 %. Aussagen über die Standzeit des Katalysators sind in der Patentschrift nicht zu finden.

Gemäß der US-A 2 018 350 kann man aromatische Dicarbonsäuren oder deren Anhydride, z.B. Phthalsäureanhydrid, mit einem reduzierenden Gas an Eisenoxidkatalysatoren, welche gegebenenfalls mit Chromverbindungen aktiviert sein können, in guten Ausbeuten zu Aldehyden hydrieren.

Nach den Angaben der EP-A 0 178 718 verwendet man in gleicher Weise aus Eisensalzlösungen hergestellte Eisenoxidkatalysatoren, um Carbonsäuren, deren Ester, Anhydride oder Salze, welche höchstens mit einem $\alpha$-Wasserstoffatom substituiert sein dürfen, zu Aldehyden zu reduzieren. Der Katalysator soll wenigstens 25 Gew.% Eisenoxid als $Fe_2O_3$ enthalten oder ganz aus Eisenoxid bestehen, wobei zur Verhinderung einer Pulverisierung ein Bindematerial beigemischt sein kann.

Die so aus salzartigen Verbindungen des Eisens hergestellten Katalysatoren haben allerdings den Nachteil, daß sie relativ schnell desaktiviert werden, wie sich aus Beispiel 1 (Vergleichsbeispiel) ergibt.

So zeigt ein entsprechend hergestellter Katalysator A (siehe Beispiele) mit der Zusammensetzung 92 % $Fe_2O_3$, 8 % $Cr_2O_3$ mit Graphit als Bindemittel anfänglich guten Umsatz und Selektivität, jedoch geht der Umsatz bei fast gleichbleibender Selektivität kontinuierlich zurück und erlischt schließlich nach 180 Betriebsstunden völlig.

Für die technische Anwendung ist es jedoch erforderlich, Mindeststandzeiten für einen Katalysator von mehreren Wochen bis zur ersten Regenerierung bzw. bis zum Katalysatorwechsel bei entsprechend hoher Produktivität zu erreichen. Bei den bisher bekannten Verfahren sind Produktivität und Standzeitverhalten unbefriedigend.

In DEFAZET - Deutsche Farben-Zeitschrift 33, 434 (1979) wird ein Verfahren zur Herstellung von röntgenamorphem $Fe_2O_3$ durch die Verbrennung von Eisencarbonyl bei Temperaturen über 500°C und Quenchung der Verbrennungsprodukte auf 250°C beschrieben. Daraus hergestelltes Eisenoxid wird als Pigment bei der Herstellung von Metallic-Lackierungen verwendet.

In Nippon Kogoku Kaisha 856 (1984) (zitiert in Chem. Abstr. 101 (1984), Zusammenfassung Nr. 239239a) wird über die Erzeugung einer $\gamma$-$Fe_2O_3$-Phase durch die Photooxidation von auf Kieselgel abgeschiedenem Eisenpentacarbonyl berichtet.

Es bestand daher die Aufgabe, einen Katalysator vorzuschlagen, der bei guter Selektivität und bei gutem Umsatz eine ausrechende Lebensdauer aufweist.

Demgemäß wurde ein Verfahren zur Herstellung von Aldehyden durch Gasphasenhydrierung von aromatischen Carbonsäuren oder aliphatischen Carbonsäuren, die in $\alpha$-Stellung zur Carboxylgruppe höchstens ein Wasserstoffatom tragen oder von Estern oder Anhydriden dieser Säuren bei Temperaturen von 300 bis 450°C und Drücken von 0,1 bis 10 bar mit Hilfe eines Eisenkatalysators gefunden, welches dadurch gekennzeichnet ist, daß der Eisenkatalysator durch die Verbrennung von Eisencarbonyl und Temperung des dabei entstandenen Eisencarbonyloxids bei 300 bis 700°C hergestellt worden ist.

Vorzugsweise verwendet man aus Eisencarbonyl hergestellte Eisenoxidkatalysatoren, welche ein Binde-

2

material enthalten, um die physikalische Struktur zu stabilisieren, oder aber man trägt die katalytisch wirksame Masse auf inerte und mechanisch feste Träger, wie Aluminiumoxid, Titanoxid, Kieselsäure, Aluminiumsilikat, Zeolithe, Magnesiumsilikat oder Aktivkohle auf. Vorzugsweise wird Aluminiumoxid verwendet.

Als Bindemittel können Übergangsmetalloxide verwendet werden; besonders bevorzugt ist dabei die Verwendung von Chromoxid. Als besonders vorteilhaft erweist sich der Zusatz von 3 bis 10 Gew. % Chromoxid. Die Menge des Bindematerials ist frei wählbar. So ist es möglich, bis zu 95 Gew.% an Bindematerial zu benutzen.

Die Katalysatorherstellung erfolgt in der Regel so, daß man Eisencarbonyl, vorzugsweise Eisenpentacarbonyl, allein oder zusammen mit anderen Stoffen in Luft oder Sauerstoff verbrennt wie es zum Beispiel in Ullmanns Encyklopädie der techn. Chemie, 4. Aufl., Bd. 10, S. 413 beschrieben ist. Das so erhaltene Carbonyleisenoxid kann man mit den anderen Komponenten mischen, eventuell trocknen und dann tablettieren. Zur Verbesserung der Fließeigenschaft des zu verformenden Materials kann ein Gleitmittel zugesetzt werden. Seine Menge richtet sich nach den Eigenschaften und der Effektivität des einzelnen Gleitmittels. Geeignete Mittel sind z.B. Graphit, höhermolekulare Fettsäuren und deren Salze. Der Katalysator kann anschließend bei Temperaturen zwischen 300 und 700°C, vorzugsweise bei 500 bis 550°C getempert werden. Soll der Katalysator als Wirbelbettkatalysator eingesetzt werden, zerkleinert man die Tabletten und siebt anschließend die Teilchen der geeigneten Korngröße heraus.

Die erfindungsgemäß zu verwendenden Katalysatoren bestehen laut Röntgenanalyse aus gut kristallisiertem $\alpha$-Fe$_2$O$_3$, dem zur Erhöhung der mechanischen Stabilität und zur Verbesserung der Verarbeitbarkeit weitere Komponenten wie Chromoxid oder Aluminiumoxid zugesetzt werden können.

Das $\alpha$-Fe$_2$O$_3$ liegt in kugelförmigen Teilchen mit einem Durchmesser von 10 bis 100 nm vor und hat dadurch eine große aktive Oberfläche und gleichzeitig, durch seine gut ausgebildete Kristallstruktur, eine gute Langzeitstabilität gegenüber Sinterung und kristallographischen Veränderungen unter den Hydrierbedingungen.

Die hohe Aktivität wird ferner durch die gegenüber konventionellen Fällungskatalysatoren (wie z.B. Katalysator A, siehe Beispiele) veränderte Porenstruktur erreicht. Während das Maximum der Porengrößenverteilung bei Katalysator A bei einem Porenradius von 20 nm liegt, befindet sich bei Carbonyleisenoxid-Katalysatoren (Katalysator C) das Maximum bei einem Porenradius von 50 nm. Durch Kombination dieser Eigenschaften gelingt es, einen sehr aktiven Katalysator, der gleichzeitig eine hohe Lebensdauer aufweist, herzustellen.

Nach einer anderen Art der Herstellung imprägniert man ein geeignetes Trägermaterial, vorzugsweise Aluminiumoxid, mit Eisencarbonyl. Jede geeignete Eisencarbonylverbindung, wie Eisenpentacarbonyl oder Dieisennonacarbonyl kann hierzu verwendet werden. Bei flüssigen Eisencarbonylen, z.B. Eisenpentacarbonyl, kann die Carbonylverbindung in reiner Form, ansonsten gelöst in einem nichtwäßrigen Lösungsmittel, wie Kohlenwasserstoffen oder Ether, nach den allgemein üblichen Imprägnierungstechniken aufgetränkt werden. Nach dem Trocknen wird der Carbonyleisenkatalysator in Gegenwart von Sauerstoff calciniert, indem man ihn langsam in einem Luft oder Sauerstoff enthaltenden Inertgasstrom auf Temperaturen zwischen 250 und 650°C, vorzugsweise zwischen 400 und 550°C, erhitzt.

Dieser Vorgang wird so oft wiederholt, bis der Katalysator die gewünschte Menge Eisenoxid enthält. Die Menge an Eisenoxid (ber. als Fe$_2$O$_3$) soll zweckmäßig 10 bis 100, vorzugsweise 20 bis 95 Gew.%, bezogen auf den gebrauchsfertigen Katalysator betragen.

Gemäß der vorliegenden Erfindung können aromatische oder aliphatische Carboxylverbindungen mit höchstens einem $\alpha$-ständigen H-Atom zur Carboxylgruppe zu den korrespondierenden Aldehyden hydriert werden. Unter Carboxylverbindungen werden dabei Carbonsäuren, deren Ester, Anhydride oder Salze verstanden. Als Salze sind vor allem die Ammoniumsalze geeignet. Aliphatische Carboxylverbindungen mit mehr als einem $\alpha$-Wasserstoffatom ergeben nur mäßige Ausbeuten.

Die Carboxylgruppe kann an einen C$_4$- bis C$_{20}$-Kohlenwasserstoffrest, bevorzugt an einen C$_5$ bis C$_{12}$-Kohlenwasserstoffrest, gebunden sein.

Da Carbonsäuren in der Regel einfacher verfügbar sind als deren Derivate, ist die Carboxylverbindung bevorzugt eine Carbonsäure. Bevorzugte Ester, welche gemäß der Erfindung eingesetzt werden können, sind C$_1$- bis C$_6$-Alkylester der obengenannten Carbonsäuren. Bevorzugte Säuren sind Benzoesäure, mit C$_1$-C$_5$-Alkylgruppen substituierte Benzoesäuren und Pivalinsäure.

Die Hydrierung wird in der Gasphase in kontinuierlicher Fahrweise durchgeführt; dabei arbeitet man bei einer Temperatur von 300 bis 450°C, vorzugsweise bei 350 bis 400°C, und einem Druck von 0,1 bis 10 bar, vorzugsweise bei atmosphärischem Druck.

Das Verfahren kann sowohl im Rohrreaktor mit stückigem Katalysatormaterial als auch in der Wirbelschicht durchgeführt werden.

Die Hydrierung wird bevorzugt mit molekularem Wasserstoff durchgeführt. Der Wasserstoff kann aber auch in situ, z.B. durch Dehydrierung von Methanol oder Cyclohexen hergestellt werden. Gegebenenfalls kann ein inertes Gas wie Stickstoff oder Argon zur Verdünnung verwendet werden. Das molare Verhältnis der carboxylischen Komponente zu Wasserstoff soll zwischen 1 : 1 und 1 : 20, bevorzugt zwischen 1 : 2 und 1 : 6 liegen.

Die Verweilzeit im Reaktor kann erheblich variiert werden. Sie liegt beim Festbettreaktor im allgemeinen zwischen 2 und 20 Sekunden, bevorzugt zwischen 6 und 14 Sekunden.

Die Reaktanten können für sich alleine oder aber in geeigneter Verdünnung mit einem inerten Gas wie Stickstoff oder Argon dem Reaktor zugeführt werden. Auch ist es möglich, die carboxylische Komponente in einem inerten Lösungsmittel wie Toluol zu lösen, um die Dosierung zu erleichtern.

Die erfindungsgemäß hergestellten Aldehyde sind wichtige industrielle Zwischenprodukte mit breiter Anwendung. Sie haben vor allem Bedeutung als Riechstoffe oder als deren Vorprodukte.

Beispiele

Katalysator A (Vergleich)

Als Katalysator A wurde ein Hochtemperatur-Konvertierungskatalysator mit der Zusammensetzung: 92 Gew.% $Fe_2O_3$, 8 Gew % $Cr_2O_3$, verwendet. Die Herstellung erfolgte durch Mischfällung von Eisen-/Chrom-Hydroxid mit Natriumhydroxid-Lösung, Filtrieren, Waschen, Trocknen, Tempern und anschließendes Tablettieren. Das Produkt entspricht dem in EP-A 0 178 718 auf S. 7 genannten Katalysator 4. Sein Litergewicht beträgt 1180 g, seine Porosität 0,25 ml/g.

Katalysator B (Vergleich)

Gefälltes Zirkoniumkarbonat wurde mit Chromsäurelösung getränkt, das Produkt getrocknet und bei 500°C getempert. Nach Zumischung von 3 % Graphit wurde das erhaltene Pulver zu 5 x 3 mm großen Tabletten verpreßt. Der Katalysator enthielt 3,1 Gew.% $Cr_2O_3$.

Katalysator C (erfindungsgemäß)

Man mischte 41 Teile Carbonyleisenoxid, 6 Teile Böhmit (Al00H) und 4 Teile Chromoxid ($Cr_2O_3$) intensiv und tablettierte unter Zusatz von 3 % Graphit. Nach Temperung bei 500°C enthalt der Katalysator 10 Gew.% $Al_2O_3$, 8 Gew.% $Cr_2O_3$ und 82 Gew.% $Fe_2O_3$. Er hat ein Litergewicht von 1060 g und eine Porosität von 0,41 ml/g.

Katalysator D (erfindungsgemäß)

Eine Mischung von 9 Teilen Carbonyleisenoxid, 13 Teilen Böhmit (Al00H) und 2 Teilen $Cr_2O_3$ wurde unter Zusatz von Wasser und 3 % Ameisensäure (bezogen auf die Trockenmasse) in einer Knetmaschine zu einer formbaren Masse vermischt. Diese Masse wurde in einer Strangpresse extrudiert, die erhaltenen Extrudate wurden getrocknet und bei 500 °C getempert. Die Formlinge wurden gemahlen und eine Fraktion zwischen 0,1 und 0,4 mm wurde ausgesiebt. Der Wirbelbettkatalysator enthält 53 % $Al_2O_3$, 39 % $Fe_2O_3$ und 8 % $Cr_2O_3$, hat eine Korngröße von 0,1 bis 0,3 mm, ein Litergewicht von 611 g und eine Porosität von 1,04 ml/g.

Beispiel 1 (Vergleichsbeispiel)

Die Umsetzung erfolgte in einem senkrecht stehenden, elektrisch beheizten Rohrreaktor mit einem inneren Durchmesser von 30 mm und einer Höhe von 450 mm. In das Reaktionsrohr füllte man 100 g (~90 ml Schüttvolumen) des Katalysators A ein und erhitzte auf 390°C. Über diesen Katalysator führt man kontinuierlich ein Gasgemisch aus Methylbenzoat und Wasserstoff im Gleichstrom. Die Verdampfung erfolgte in einem eigens vorgeschalteten ebenfalls elektrisch beheizten Verdampfer. Die Einspeisegeschwindigkeit betrug 0,18 mol Methylbenzoat pro Stunde und 1,8 mol Wasserstoff pro Stunde. Das Reaktionsgemisch wurde mit einem Wasserkühler kondensiert und gaschromatographisch analysiert. Als Nebenprodukte fand man anfangs 12 % Toluol, 4 % Benzol, 1 % Benzylalkohol und 3 % nicht identifizierte Nebenprodukte.

Der anfängliche Umsatz betrug 75 %, fiel dann aber laufend ab, betrug nach 180 Stunden nur noch 50 % und erlosch schließlich ganz. Die Selektivität betrug in den ersten zehn Stunden 80 %, ging aber auf gleichbleibend 70 % zurück.

Beispiel 2 (Vergleichsbeispiel)

Man verfuhr wie in Beispiel 1 und setzte Katalysator B (100 ml) ein. Den Rohrreaktor heizte man auf 415°C und leitete bei dieser Temperatur pro Stunde ein Gasgemisch aus 27,2 g (0,2 mol) Methylbenzoat und 1,21 g (0,6 mol) Wasserstoff über den Katalysator.

Die Selektivität für Benzaldehyd betrug nach 10 Betriebsstunden 60 % bei 20 % Umsatz.

Beispiel 3 (erfindungsgemäß)

Man verfuhr wie in Beispiel 1 und setzte den Katalysator C (90 ml Schüttvolumen) ein. Den Rohrreaktor heizte man auf 360°C und leitete bei dieser Temperatur pro Stunde ein Gasgemisch aus 24,5 g (0,18 mol) Methylbenzoat und 1,09 g (0,54 mol) Wasserstoff über den Katalysator. Die Verweilzeit (bezogen auf Schüttvolumen) betrug 8,56 sec. Mit einem Wasserkühler kondensierte man hinter dem Reaktor das Produktgemisch. Die Selektivität betrug bei über 800 Betriebsstunden gleichbleibend 86 % bei einem ebenfalls gleichbleibenden Umsatz von 72 %. Der Katalysator zeigte bis zu diesem Zeitpunkt keinerlei Desaktivierungserscheinungen.

Als Nebenprodukt entstanden lediglich 8 % Toluol, 3 % Benzol und 3 % nicht identifizierte Nebenprodukte.

Beispiel 4 (erfindungsgemäß)

Man verfuhr wie in Beispiel 3 und setzte Katalysator C ein. Den Rohrreaktor heizte man auf 420°C und leitete bei dieser Temperatur pro Stunde ein Gasgemisch aus 27,9 g (0,27 mol) Pivalinsäure und 0,6 mol Wasserstoff über den Katalysator.

Die Selektivität betrug nach über 400 Betriebsstunden gleichbleibend 85 % bei ebenfalls gleichbleibendem Umsatz von 83 %. Der Katalysator zeigte bis zu diesem Zeitpunkt wiederum keinerlei Desaktivierungserscheinungen.

Beispiel 5

Man verfuhr wie in Beispiel B und setzte Katalysator C (90 ml) ein. Den Rohrreaktor heizte man auf 390°C und leitete bei dieser Temperatur pro Stunde ein Gasgemisch aus 18,82 g (0,1 mol) p-tert.-Butylbenzoesäure und 0,64 g (0,32 mol) Wasserstoff über den Katalysator. Die Selektivität für p-tert.-Butylbenzaldehyd betrug nach 100 Betriebsstunden 70 % bei 85 % Umsatz.

Die Eigenschaften der Katalysatoren A bis C sowie die Ergebnisse bei deren Verwendung sind in der folgenden Tabelle vergleichend zusammengestellt.

Tabelle

| KATALYSATOR | A | B | C |
|---|---|---|---|
| Zusammensetzung | 92 % $Fe_2O_3$ | 96,6 % $ZrO_2$ | 82 % $Fe_2O_3$ |
| | 8 % $Cr_2O_3$ | 3,1 % $Cr_2O_3$ | 10 % $Al_2O_3$ |
| | | | 8 % $Cr_2O_3$ |
| Herstellung | Fällung gem. | $CrO_3$-Lsg.auf- | aus Carbonyl- |
| | EP 178 718 | getr. $ZrOCO_3$ | eisenoxid |
| Porosität (ml/g) | 0,25 | 0,12 | 0,41 |
| Litergewicht (g) | 1180 | 1780 | 1060 |
| Oberfl. $m^2/g$ | 70 | 5,8 | 49 |
| Porenrad. nm (Max. d. Vert.) | 20 | | 50 |
| Härte $kg/cm^2$ | 250 | 94 | 402 |

| BEISPIEL | 1 | 2 | 3 | 4 | 5 |
|---|---|---|---|---|---|
| Edukt | Methylbenzoat | Methyl-benzoat | Methylbenzoat | Pivalin-säure | 4-t-Butyl-benzoes. |
| Katalysator | A | B | C | C | C |
| Temp. ($^0$C) | 390 | 415 | 360 | 420 | 390 |
| LHSV(mol/h·l) (liquid hourly space velocity) | 2 | 2 | 2 | 3 | 1,22 |
| Umsatz | 75 % | 20 % | 72 % | 83 % | 85 % |
| Selektiv. | 80 % | 60 % | 86 % | 85 % | 70 % |
| Nebenprod. | Toluol,Benzol, Benzylalkohol | | Toluol,Benzol | | |
| Lebensdauer | 180 h bis Umsatz auf 50 % fiel | ca. 10 h | > 800 h | > 400 h | |

**Patentansprüche**

1. Verfahren zur Herstellung von Aldehyden durch Gasphasenhydrierung von aromatischen Carbonsäuren oder aliphatischen Carbonsäuren, die in $\alpha$-Stellung zur Carboxylgruppe höchstens ein Wasserstoffatom tragen, oder von Estern oder Anhydriden dieser Säuren bei Temperaturen von 300 bis 450°C und Drücken von 0,1 bis 10 bar mit Hilfe eines Eisenkatalysators, dadurch gekennzeichnet, daß der Eisenkatalysator durch die Verbrennung von Eisencarbonyl und Temperung des dabei entstandenen Carbonyleisenoxids bei 300 bis 700°C hergestellt worden ist.

2. Verfahren nach Anspruch 1, dadurch gekennzeichnet, daß der Katalysator durch Verbrennen des Eisencarbonyls in Luft oder Sauerstoff, Mischen des erhaltenen Eisenoxids mit Hilfsmitteln und Tempern hergestellt ist.

3. Verfahren nach Anspruch 1, dadurch gekennzeichnet, daß der Katalysator durch Tränken eines Trägers mit Eisencarbonyl, Trocknen und Calcinieren in einem Luft oder Sauerstoff enthaltenden Strom hergestellt ist.

4. Verfahren nach Anspruch 1, dadurch gekennzeichnet, daß man als Ausgangsstoff Benzoesäure, Methylbenzoat, p-tert.-Butylbenzoesäure oder Pivalinsäure verwendet.

## Claims

1. A process for preparing an aldehyde by gas phase hydrogenation of an aromatic or aliphatic carboxylic acid having not more than one hydrogen atom alpha to the carboxyl group, or of an ester or anhydride thereof, at from 300 to 450°C under from 0.1 to 10 bar by means of an iron catalyst prepared by burning iron carbonyl and heat-treating the resulting carbonyl iron oxide at from 300 to 700°C.

2. A process as claimed in claim 1, wherein the catalyst has been prepared by burning iron carbonyl in air or oxygen, mixing the resulting iron oxide with an assistant, and heat-treating the mixture.

3. A process as claimed in claim 1, wherein the catalyst has been prepared by impregnating a carrier with iron carbonyl, drying, and calcining in an air- or oxygen-containing stream.

4. A process as claimed in claim 1, wherein the starting material used is benzoic acid, methyl benzoate, p-tert-butylbenzoic acid or pivalic acid.

## Revendications

1. Procédé de préparation d'aldéhydes par hydrogénation en phase gazeuse d'acides carboxyliques aromatiques ou d'acides carboxyliques aliphatiques qui portent au maximum un atome d'hydrogène en position $\alpha$ par rapport au groupement carboxyle, ou d'esters ou d'anhydrides de ces acides, à des températures de 300 à 450°C et sous des pressions de 0,1 à 10 bar, à l'aide d'un catalyseur à base de fer, caractérisé en ce que le catalyseur à base de fer a été préparé par combustion de fer-carbonyle et recuisson de l'oxyde de fer-carbonyle ainsi obtenu à une température de 300 à 700°C,

2. Procédé selon la revendication 1, caractérisé en ce que le catalyseur est prépare par combustion du fercarbonyle dans de l'air ou de l'oxygène, mélange de l'oxyde de fer obtenu avec des adjuvants et recuisson.

3. Procédé selon la revendication 1, caractérisé en ce que le catalyseur est préparé par imprégnation d'un support avec du fer-carbonyle, séchage et calcination dans un courant contenant de l'air ou de l'oxygène.

4. Procédé selon la revendication 1, caractérisé en ce qu'on utilise, comme substance de départ, de l'acide benzoïque, du benzoate de méthyle, de l'acide p-tert.-butylbenzoïque ou de l'acide pivalique.